# EUROPÄISCHE PATENTSCHRIFT

(11) **EP 1 750 571 B1**
(45) Veröffentlichungstag und Bekanntmachung des Hinweises auf die Patenterteilung: **08.04.2009**
(21) Anmeldenummer: 05745224.5
(22) Anmeldetag: 25.05.2005
(51) Int. Cl.: A61B 1/267

(54) **LARYNGOSKOP**
LARYNGOSCOPE
LARYNGOSCOPE

(30) Priorität: 03.06.2004 DE 102004028428
(43) Veröffentlichungstag der Anmeldung: 14.02.2007
(73) Patentinhaber: Karl Storz GmbH & Co. KG, 78532 Tuttlingen (DE)
(72) Erfinder: HENSLER, Fritz, 78579 Neuhausen (DE)
(74) Vertreter: Heuckeroth, Volker
(86) Internationale Anmeldenummer: PCT/EP2005/005623
(87) Internationale Veröffentlichungsnummer: WO 2005/117686

(56) Entgegenhaltungen:
- DE-U1- 8 812 092
- US-A- 4 947 896
- US-A1- 2001 023 312

## Beschreibung

Die Erfindung betrifft ein Laryngoskop, mit einem Laryngoskopspatel, einem Handgriff sowie mit einer Beleuchtungseinrichtung zum Ausleuchten des Mund- bzw. Rachenraumes, wobei die Beleuchtungseinrichtung zumindest ein erstes Licht abstrahlendes Element aufweist, das am Laryngoskopspatel angeordnet ist und im Betrieb einen Lichtkegel mit einem Öffnungswinkel abstrahlt, und wobei die Beleuchtungseinrichtung zumindest ein zweites Licht abstrahlendes Element aufweist, das ebenfalls am Laryngoskopspatel angeordnet ist.

Ein derartiges Laryngoskop ist aus dem Dokument US 4947896 A bekannt.

Laryngoskope sind medizinische Instrumente, mit denen der Kehlkopf (Larynx) endoskopisch inspiziert werden kann. Im Rahmen einer laryngoskopischen Inspektion wird der Laryngoskopspatel in den Mund des Patienten eingeführt und dessen distales Ende bis in die Nähe des Kehlkopfes vorgeschoben.

Beim Einführen des Laryngoskopspatels in den Mund- und Rachenraum muss der behandelnde Arzt sehr vorsichtig vorgehen, um Verletzungen im Rachenraum, insbesondere im Bereich des Kehlkopfes, zu vermeiden. Der behandelnde Arzt benötigt daher eine sehr gute Sicht in den Mund- bzw. Rachenraum. Eine gute Sicht im Mund- und Rachenraum setzt jedoch eine ausreichende Ausleuchtung desselben voraus, wozu ein Laryngoskop üblicherweise mit einer Beleuchtungseinrichtung ausgestattet ist.

Es sind bislang Laryngoskope mit verschiedensten Arten von Beleuchtungseinrichtungen bekannt.

Im Sinne der vorliegenden Erfindung ist als Licht abstrahlendes Element ein Element zu verstehen, von dem schließlich das Licht in den Mund- bzw. Rachenraum abgestrahlt wird.

Der Begriff "Lichtkegel" ist hinsichtlich der Form der Einhüllenden ganz allgemein zu verstehen und ist nicht auf den mathematischen Kegelbegriff beschränkt.

Ein aus dem Dokument WO-A-02/071930 bekanntes Laryngoskop weist als Licht abstrahlendes Element eine Leuchtdiode (LED) auf, die am Laryngoskopspatel angeordnet ist, wobei in jenem Dokument es jedoch als bevorzugt angesehen ist, die Leuchtdiode im Handgriff anzubringen und das von der Leuchtdiode emittierte Licht über einen Lichtleiter in den distalen Bereich des Laryngoskopspatels zu führen und dort abzustrahlen, um den Mund- bzw. Rachenraum auszuleuchten.

Die Verwendung von Leuchtdioden als Lichtquellen bzw. Licht abstrahlende Elemente in Laryngoskopen ist durch die technische Weiterentwicklung von Leuchtdioden, insbesondere Weißlicht-Leuchtdioden, möglich geworden. Insbesondere sind heute Leuchtdioden erhältlich, die eine hohe Lichtausbeute gewährleisten.

Das aus dem oben genannten Dokument US 4947896 A bekannte Laryngoskop weist am Laryngoskopspatel zwei Lichtquellen auf, wobei über die Abstrahlcharakteristik dieser beiden Lichtquellen in dem Dokument nichts ausgesagt wird.

Aus dem Dokument DE 8812092 U ist ein Laryngoskop bekannt, das einen als Lichtleiter oder als Stromzuführung für eine Lichtquelle am Laryngoskopspatel ausgebildeten Kanal aufweist. Das Lichtaustrittsende des Kanals kann optische Mittel zur divergierenden Aufspreizung des austretenden Lichtstrahlenbündel aufweisen.

Bei Laryngoskopen älterer Bauweisen wurden Glühbirnen für die Beleuchtungseinrichtung verwendet, die entweder im Handgriff angeordnet werden, wobei dann wiederum das von der Glühbirne emittierte Licht über einen Lichtleiter in den Laryngoskopspatel geführt und von dort abgestrahlt wird, oder die Glühbirne wird unmittelbar im Laryngoskopspatel angeordnet, während die Spannungsversorgung der Glühbirne über eine im Handgriff angeordnete Batterie erfolgt, die über elektrische Leitungen mit der Glühbirne in dem Laryngoskopspatel verbunden ist.

Ein Laryngoskop der zuletzt genannten Art ist beispielsweise in dem Dokument DE-A-42 43 790 offenbart.

Das Dokument DE-A-102 13 919 offenbart ein medizinisches Instrument, wie beispielsweise Wundhaken, Gehirnspatel, Muskelspreizer, Scheidenspekulum und dergleichen, das ein Arbeitsteil zum Offenhalten einer Eingriffstelle aufweist, wobei das Arbeitsteil distalseitig eine Beleuchtungseinrichtung zur Beleuchtung der Eingriffstelle aufweist. Die Beleuchtungseinrichtung dieses bekannten Instruments weist zumindest eine Weißlicht emittierende Leuchtdiode auf, die über eine zumindest teilweise innerhalb des Arbeitsteils angeordnete, elektrisch leitende Verbindung mit einer außerhalb des Arbeitsteils befindlichen Stromquelle verbunden ist. Die Beleuchtungseinrichtung kann bei diesem bekannten Instrument vorzugsweise aus mehreren arrayartig angeordneten, Weißlicht emittierenden Leuchtdioden zusammengesetzt sein.

Mit dem Vorsehen einer Mehrzahl von Licht abstrahlenden Elementen bzw. Leuchtmitteln, wie Leuchtdioden, am Laryngoskopspatel lässt sich die Gesamtlichtstärke entsprechend der Anzahl an Licht abstrahlenden Elementen anpassen. Dies führt jedoch nicht zwangsläufig zu einer verbesserten Ausleuchtung des gesamten Mund- bzw. Rachenraumes und damit einer verbesserten Orientierung und Sicht des Arztes. Eine bloße Erhöhung der Lichtstärke kann sogar dazu führen, dass der Arzt bei der Inspektion des Kehlkopfes geblendet werden kann.

Der Erfindung liegt daher die Aufgabe zugrunde, ein Laryngoskop der eingangs genannten Art dahingehend weiterzubilden, dass es eine verbesserte Ausleuchtung und damit verbesserte Inspektion des Mund- bzw. Rachenraumes im Rahmen der Laryngoskopie ermöglicht.

Erfindungsgemäß wird diese Aufgabe hinsichtlich des eingangs genannten Laryngoskops dadurch gelöst, dass das zumindest eine zweite Licht abstrahlende Element einen zweiten Lichtkegel mit einem zweiten Öffnungswinkel abstrahlt, der größer ist als der öffnungswinkel des Lichtkegels des ersten Licht abstrahlenden Elements, und dass die Licht abstrahlenden Elemente so angeordnet sind, das die Lichtkegel teilweise einander überlagert sind.

Auch die vorliegende Erfindung geht zunächst davon aus, dass durch Erhöhung der Anzahl an Licht abstrahlenden Elementen am Laryngoskopspatel die Gesamtlichtstärke erhöht werden kann. Im Unterschied zu den aus dem Stand der Technik bekannten Laryngoskopen wird das "Mehr" an Lichtstärke jedoch gleichmäßiger im Mund- bzw. Rachenraum verteilt, indem nämlich zumindest zwei Licht abstrahlende Elemente am Laryngoskopspatel vorhanden sind, die das Licht mit unterschiedlichen Öffnungswinkeln abstrahlen. Mit dem ersten Licht abstrahlenden Element kleineren Öffnungswinkels wird die Tiefe der Ausleuchtung verbessert, während mit dem zweiten Licht abstrahlenden Element größeren Öffnungswinkels die Breite der Ausleuchtung verbessert wird. Anstatt wie im Stand der Technik mehr Licht durch mehr Licht abstrahlende Elemente in Richtung nur einer Achse zu konzentrieren, wird bei der erfindungsgemäßen Ausgestaltung des Laryngoskops eine Verbesserung der Ausleuchtung in zwei Achsrichtungen erreicht, was auch ein Blenden des Arztes durch eine übermäßige Lichtstärke in einer Achsrichtung vermeidet. Mit anderen Worten trägt die Beleuchtungseinrichtung des erfindungsgemäßen Laryngoskops der Anatomie des Mund- bzw. Rachenraumes besser Rechnung als die Beleuchtungseinrichtungen der bekannten Laryngoskope.

Die Licht abstrahlenden Elemente sind dabei so angeordnet, dass die Lichtkegel teilweise einander überlagert sind.

Hierbei ist von Vorteil, dass es einen Lichtkegelbereich gibt, in dem sich die Lichtstärkeverteilungen der beiden Licht abstrahlende Elemente teilweise addieren und somit eine höhere Gesamtlichtstärke im Überlagerungsbereich ergeben. Zweckmäßig und vorteilhaft werden die Licht abstrahlenden Elemente so angeordnet, dass sich der Überlagerungsbereich in etwa geradliniger Verlängerung der Längsrichtung des Laryngoskopspatels befindet. Weiter vorzugsweise sind die Licht abstrahlenden Elemente im distalen Bereich des Laryngoskopspatels nebeneinander auf etwa gleicher Höhe bzgl. der Längsrichtung des Laryngoskopspatels angeordnet.

In einer weiteren bevorzugten Ausgestaltung ist eine erste Hauptabstrahlrichtung des ersten Licht abstrahlenden Elements parallel zu einer zweiten Hauptabstrahlrichtung des zweiten Licht abstrahlenden Elements.

Wenn die zumindest zwei Licht abstrahlenden Elemente nahe nebeneinander angeordnet sind, können die beiden zuvor genannten Hauptabstrahlrichtungen sogar nahezu zusammenfallen. Der Vorteil der genannten Maßnahme in Verbindung mit der zuvor genannten Maßnahme besteht insbesondere darin, dass ein großer Überlagerungsbereich der zumindest zwei Lichtkegel erreicht werden kann.

In einer weiteren bevorzugten Ausgestaltung ist der zweite Öffnungswinkel um etwa das 1,5-fache bis 4-fache größer als der erste Öffnungswinkel.

Hierbei ist von Vorteil, dass sich die zumindest zwei Lichtkegel hinsichtlich ihrer Öffnungswinkel deutlich unterscheiden, wodurch sowohl die Ausleuchtung in der Tiefe als auch die Ausleuchtung in der Breite noch weiter verbessert sind.

In einer bevorzugten praktischen Ausgestaltung liegt der erste Öffnungswinkel im Bereich von etwa 10° bis etwa 30° und der zweite Öffnungswinkel im Bereich von etwa 40° bis etwa 70°.

In einem bevorzugten Beispiel kann der erste Öffnungswinkel etwa 25° für die Tiefenausleuchtung und der zweite Öffnungswinkel etwa 60° für die Breitenausleuchtung betragen.

In einer weiteren bevorzugten Ausgestaltung sind das erste Licht abstrahlende Element und das zumindest eine zweite Licht abstrahlende Element Lichtquellen.

Es ist zwar im Rahmen der Erfindung auch denkbar, dass das erste Licht abstrahlende Element und das zweite Licht abstrahlende Element die Austrittsflächen von Lichtleitern sind. Demgegenüber hat die Ausgestaltung der zumindest zwei Licht abstrahlenden Elemente als Lichtquellen den Vorteil, dass Übertragungsverluste durch längere Lichtleiter vermieden werden. Von den Lichtquellen, die vorzugsweise im distalen Bereich des Laryngoskopspatels angeordnet sind, wird das Licht dann direkt ohne Übertragungsstrecke durch einen Lichtleiter in den Mund- bzw. Rachenraum abgestrahlt.

In einer besonders bevorzugten Ausgestaltung sind das erste Licht abstrahlende Element und das zumindest eine zweite Licht abstrahlende Element jeweils zumindest eine Leuchtdiode, insbesondere eine Weißlicht-Leuchtdiode.

Leuchtdioden mit unterschiedlichem Öffnungswinkel der von ihnen abgestrahlten Lichtkegel sind derzeit erhältlich und eignen sich daher besonders zur Verwendung in der vorliegenden Erfindung, zumal solche Leuchtdioden kostengünstig sind und heutzutage, wie bereits eingangs erwähnt, mit hohen Lichtstärken erhältlich sind. Darüber hinaus können solche Leuchtdioden auch ohne großen Aufwand in den Laryngoskopspatel eingebaut werden, beispielsweise in eine genormte Glühbirnenfassung, die bei Laryngoskopspateln bisheriger Bauart, bei denen Glühbirnen für die Beleuchtungseinrichtung verwendet wurden, verwendet werden.

In diesem Zusammenhang ist bevorzugt, wenn für die erste Leuchtdiode und die zumindest eine zweite Leuchtdiode eine gemeinsame Spannungsversorgung vorgesehen ist.

Hierbei ist von Vorteil, dass gegenüber herkömmlichen Laryngoskopen mit nur einer Lichtquelle kein wesentlich erhöhter Platzbedarf für die Spannungsversorgung erforderlich ist.

In einer weiteren bevorzugten Ausgestaltung weist die Spannungsversorgung zumindest eine im Handgriff angeordnete Batterie auf.

Die Verwendung einer Batterie als Spannungsversorgung für die zumindest zwei Leuchtdioden hat den Vorteil, dass das Laryngoskop als Ganzes autonom ist, d.h. keine externe Spannungsversorgung und damit Kabel, die bei dem Gebrauch des Laryngoskops stören würden, benötigt werden.

Die zumindest eine erste Leuchtdiode und die zumindest eine zweite Leuchtdiode können in Parallelschaltung oder Serienschaltung an die gemeinsame Spannungsversorgung angeschlossen sein, wobei im Falle der Parallelschaltung zur Spannungsbegrenzung entsprechende Vorwiderstände an den Leuchtdioden vorgesehen werden können, und im Falle einer Serienschaltung kann eine elektronische Treiberschaltung vorgesehen sein, um die gegebenenfalls von der oder den Batterien bereitgestellte Spannung zu erhöhen, um die Durchbruchspannung der zumindest zwei Leuchtdioden zu erreichen.

In einer weiteren bevorzugten Ausgestaltung ist für die zumindest eine Batterie eine Batteriespannung-Überwachungsschaltung vorhanden.

Hierbei ist von Vorteil, dass rechtzeitig ein Zustand erkannt werden kann, in dem die Spannung zum Betreiben der zumindest zwei Leuchtdioden nicht mehr ausreicht.

Dabei ist es insbesondere bevorzugt, wenn die Batteriespannung-Überwachungsschaltung ein Signal erzeugt, wenn die Batteriespannung einen vorgegebenen Mindestwert unterschreitet, wobei das Signal von zumindest einer der zwei Leuchtdioden erzeugt wird.

Hierbei ist von Vorteil, dass zumindest eine der beiden Leuchtdioden zusätzlich noch die Funktion einer Anzeige einer zu geringen Batteriespannung übernimmt, so dass vorteilhafterweise keine weitere Leuchtdiode zur Anzeige verwendet werden muss, wie dies beispielsweise bei dem aus dem Dokument WO-A-02/071930 bekannten Laryngoskop der Fall ist.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: ein Laryngoskop in Seitenansicht;
- Fig. 2: den Laryngoskopspatel des Laryngoskops in Fig. 1 in vergrößertem Maßstab in Alleinstellung, teilweise mit Aufbrechungen;
- Fig. 3: den Laryngoskopspatel in Fig. 2 in Draufsicht, teil- weise mit Aufbrechungen;
- Fig. 4: ein Blockschaltbild des Laryngoskops in Fig. 1; und
- Fig. 5: ein zu Fig. 4 alternatives Blockschaltbild einer bei dem Laryngoskop in Fig. 1 realisierbaren Variante.

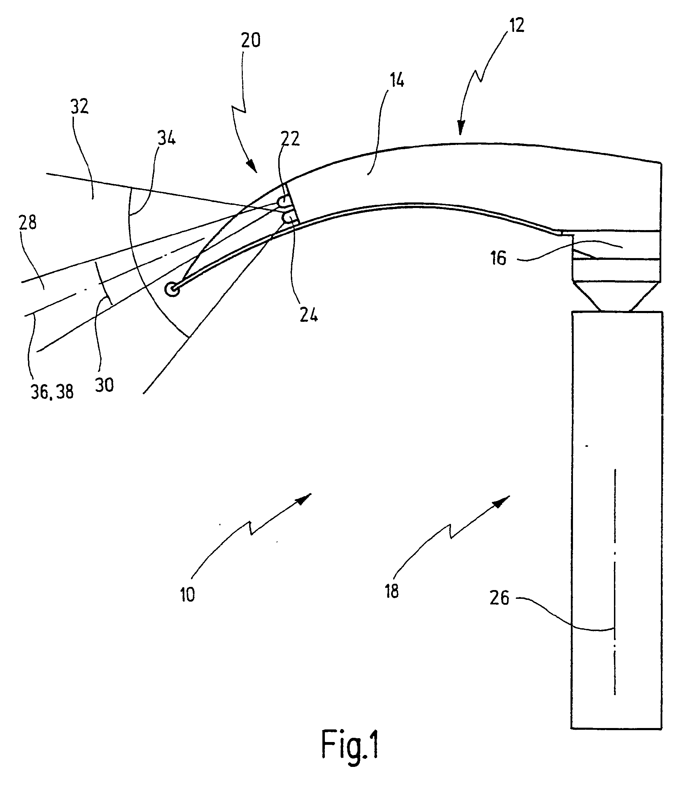

In Fig. 1 ist ein mit dem allgemeinen Bezugszeichen 10 versehenes Laryngoskop dargestellt.

Das Laryngoskop 10 wird bei medizinischen bzw. chirurgischen diagnostischen Verfahren beispielsweise zur Inspektion des Kehlkopfes verwendet.

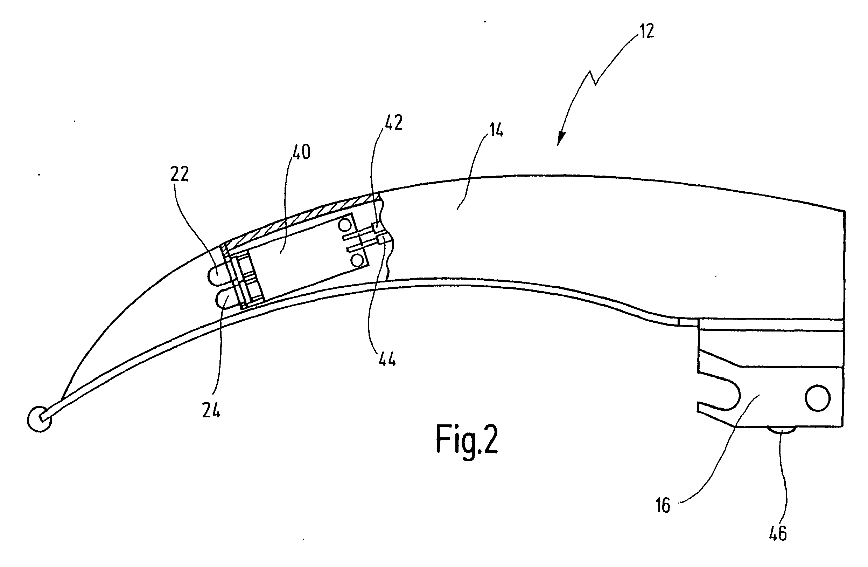

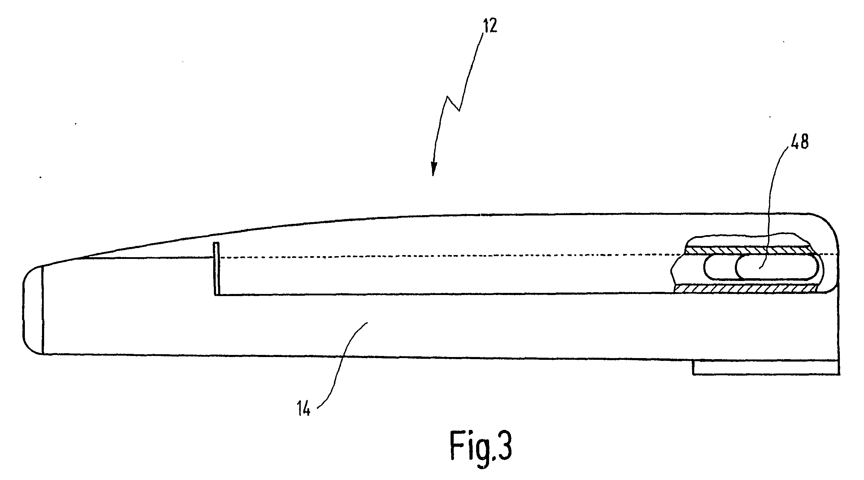

Das Laryngoskop 10 weist einen Laryngoskopspatel 12 auf, der in Fig. 2 und 3 in Alleinstellung gezeigt ist. Der Laryngoskopspatel 12 weist ein Spatelblatt 14 sowie einen Spatelkopf 16 auf. Über den Spatelkopf 16 ist der Laryngoskopspatel 12 mit einem Handgriff 18 abnehmbar verbunden.

Das Laryngoskop 10 weist zur Ausleuchtung des Mund- bzw. Rachenraums eine Beleuchtungseinrichtung 20 auf.

Die Beleuchtungseinrichtung 20 weist ein erstes Licht abstrahlendes Element 22 und ein zweites Licht abstrahlendes Element 24 auf, wobei weitere Licht abstrahlende Elemente vorgesehen sein können.

Das erste Licht abstrahlende Element 22 und das zweite Licht abstrahlende Element 24 sind am Laryngoskopspatel 12, genauer gesagt am Spatelblatt 14, in dessen distalem Bereich angeordnet.

Die Licht abstrahlenden Elemente 22 und 24 sind in dem Ausführungsbeispiel in Richtung einer Handgriffachse 26 hintereinander angeordnet, wobei jedoch auch die Anordnung um 90° verdreht am Laryngoskopspatel 12 vorgesehen sein könnte.

Im Betrieb der Beleuchtungseinrichtung 20 strahlt das erste Licht abstrahlende Element 22 einen ersten Lichtkegel 28 mit einem ersten Öffnungswinkel 30 ab. Das zweite Licht abstrahlende Element 24 strahlt im Betrieb der Beleuchtungseinrichtung 20 einen zweiten Lichtkegel 32 mit einem zweiten Öffnungswinkel 34 ab, wobei der zweite Öffnungswinkel 34 größer ist als der erste Öffnungswinkel 30 des ersten Lichtkegels 28.

Die Licht abstrahlenden Elemente 22 und 24 sind nahe beieinander angeordnet, so dass die Lichtkegel 28 und 32 teilweise einander überlagert sind.

Eine erste Hauptabstrahlrichtung 36 des ersten Licht abstrahlenden Elements 22 ist parallel zu einer zweiten Hauptabstrahlrichtung 38 des zweiten Licht abstrahlenden Elements 24, wobei die beiden Hauptabstrahlrichtungen 36, 38 vollständig oder nahezu zusammenfallen, wie in Fig. 1 zeichnerisch veranschaulicht ist.

Der zweite Öffnungswinkel 34 des zweiten Lichtkegels 32 beträgt im vorliegenden Ausführungsbeispiel etwa 60°, und der Öffnungswinkel 30 des ersten Lichtkegels 28 etwa 25°, so dass der zweite Öffnungswinkel 34 um etwa das 2,5-fache größer ist als der erste Öffnungswinkel 30.

Mit dem ersten Lichtkegel 28 kleineren Öffnungswinkels 30 kann somit der Mund- bzw. Rachenraum in der Tiefe sehr gut ausgeleuchtet werden, während mit dem Lichtkegel 32 eine Ausleuchtung des Mund- bzw. Rachenraumes in der Breite sehr gut möglich ist, wodurch sich insgesamt eine sehr gute und gleichmäßige Ausleuchtung des gesamten Mund- bzw. Rachenraumes ergibt, ohne dass das Laryngoskop 10 dazu im in den Mund eingeführten Zustand verschieden orientiert werden muss.

Das erste Licht abstrahlende Element 22 und das zweite Licht abstrahlende Element 24 sind Lichtquellen, d.h. die Lichterzeugung findet unmittelbar in den Licht abstrahlenden Elementen 22 und 24 statt.

Die Licht abstrahlenden Elemente 22 und 24 sind Leuchtdioden, insbesondere Weißlicht-Leuchtdioden.

Während zuvor beschrieben wurde, dass der Öffnungswinkel 30 des ersten Licht abstrahlenden Elements 22 etwa 25° beträgt, können auch Leuchtdioden verwendet werden, die einen Lichtkegel mit einem Öffnungswinkel im Bereich von etwa 10° bis etwa 30° abstrahlen. Für das zweite Licht abstrahlende Element 24 können auch solche Leuchtdioden verwendet werden, die einen Lichtkegel mit einem Öffnungswinkel im Bereich von etwa 40° bis etwa 70° abstrahlen.

Das Verhältnis zwischen den Öffnungswinkeln 34 und 30 kann insbesondere im Bereich von 1,5 bis 4 liegen.

Wie in Fig. 2 dargestellt ist, sind die Licht abstrahlenden Elemente 22 und 24 in Form der Leuchtdioden an einer Fassung 40 am Spatelblatt 14 in dieses integriert montiert. Die Fassung 40 kann beispielsweise eine herkömmliche Glühbirnenfassung oder auch eine Platine sein, an der die Leuchtdioden dann geeignet befestigt sind.

Zwei Stromzuführungen 42 und 44 für den Plus-Pol und den MinusPol führen von der Fassung 40 in den proximalen Bereich des Laryngoskopspatels 12, wobei die Stromzuführung 42 (Plus-Pol) am Spatelkopf 16 mit einem Kontakt 46 verbunden ist.

Im proximalen Bereich des Laryngoskopspatels 12 ist eine entsprechende Durchführung 48 vorgesehen, wie in Fig. 3 dargestellt ist. In Fig. 3 sind die Stromzuführungen 42 und 44 nicht dargestellt.

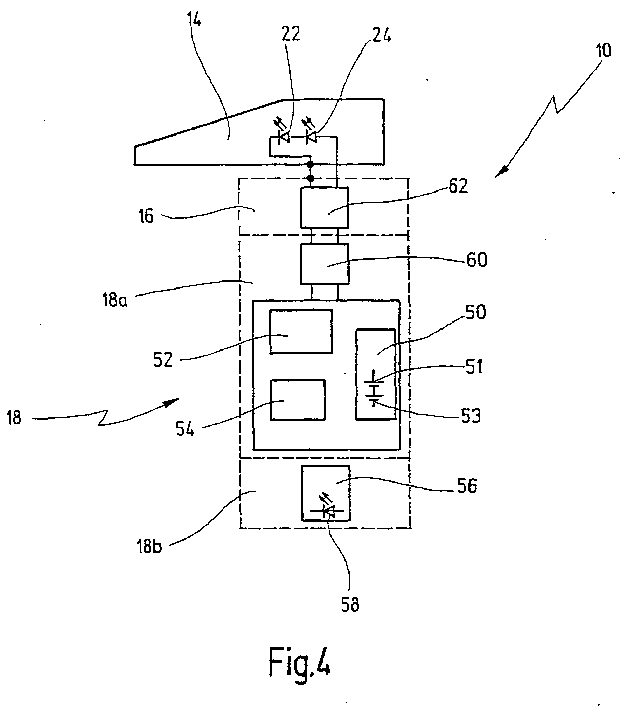

Mit Bezug auf Fig. 4 wird nachfolgend insbesondere die elektrische Schaltung der Leuchtdioden sowie die Aspekte der Spannungsversorgung der beiden Leuchtdioden beschrieben.

In Fig. 4 sind das Spatelblatt 14, der Spatelkopf 16 und der Handgriff 18 schematisch dargestellt, wobei der Handgriff 18 in ein Handgriffhauptteil 18a und ein Handgriffendteil 18b unterteilt dargestellt sind. Das Handgriffendteil 18b ist als Schraubdeckel ausgeführt und kann vom Handgriffhauptteil 18a entsprechend abgenommen werden.

Für die Licht abstrahlenden Elemente 22 und 24 in Form der Leuchtdioden ist eine gemeinsame Spannungsversorgung 50 vorgesehen, die im vorliegenden Ausführungsbeispiel durch zwei 3 V-Batterien 51, 53 gebildet ist, so dass eine Gesamtspannung von 6 V anliegt.

Die Spannungsversorgung 50 ist, wie in Fig. 4 dargestellt ist, im Handgriffhauptteil 18a angeordnet.

Die die Licht abstrahlenden Elemente 22, 24 bildenden Leuchtdioden sind bei dieser Schaltungsvariante in Reihe geschaltet. Dadurch liegt die Durchbruchspannung der Leuchtdioden gegebenenfalls zusammen etwas über der vorhandenen Spannungsversorgung von 6 V.

Um sicherzustellen, dass die Durchbruchspannung der Leuchtdioden in diesem Falle erreicht wird, ist eine Ansteuerelektronik in Form einer Treiberschaltung 52 im Handgriff 18 angeordnet, die als integrierte Schaltung ausgeführt ist.

Des Weiteren ist im Handgriff eine Batteriespannung-Überwachungsschaltung 54 vorhanden, die die Spannung der Batterien 51, 53 überwacht.

Eine Warneinrichtung 56 steht mit der Batteriespannung-Überwachungsschaltung in Verbindung und erzeugt ein Signal, wenn die Batteriespannung einen vorbestimmten Mindestwert unterschreitet. Ein solches Signal wird beispielsweise von einer zusätzlichen Leuchtdiode 58 erzeugt, die beispielsweise im Falle des Unterschreitens des vorbestimmten Spannungsmindestwertes blinkt.

Anstatt einer zusätzlichen Leuchtdiode, wie die Leuchtdiode 58, kann das Signal jedoch auch durch eine der beiden die Licht abstrahlenden Elemente 22, 24 bildenden Leuchtdioden erzeugt werden.

Des Weiteren ist am Handgriff 18 ein Ein-/Aus-Schalter 60 sowie ein Kopfanschluss 62 vorgesehen, der die elektrische Verbindung zwischen dem Spatelkopf 16 und dem Handgriff 18 herstellt.

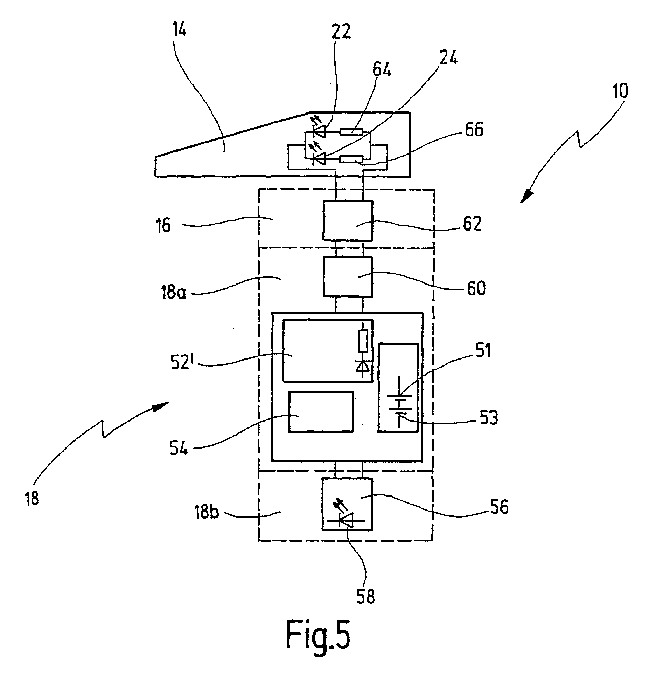

In Fig. 5 ist ein gegenüber Fig. 4 geringfügig abgewandeltes Schaltschema für die beiden Licht abstrahlenden Elemente 22 und 24 in Form der Leuchtdioden dargestellt.

Im Unterschied zu der Schaltungsvariante in Fig. 4 sind die die Licht abstrahlenden Elemente 22, 24 bildenden Leuchtdioden parallelgeschaltet. Dadurch liegt die benötigte Durchbruchspannung für die beiden Leuchtdioden unter den durch die Batterien 51 und 53 bereitgestellten 6 V. Die Treiberschaltung 52 in Fig. 4 ist daher nicht erforderlich. Allerdings sollten die Leuchtdioden der Licht abstrahlenden Elemente 22, 24 mit eigenen Vorwiderständen 64, 66 betrieben werden, um die Leuchtdioden vor zu hohen Strömen zu bewahren und um eine einigermaßen gleichmäßige Lichtverteilung zwischen den beiden Leuchtdioden zu erreichen.

Anstelle der Treiberschaltung 52 in Fig. 4 ist nun ein Vorwiderstand mit Diode (Bezugszeichen 52') vorgesehen.

Im Übrigen stimmt die Schaltung in Fig. 5 mit der Schaltung in Fig. 4 überein.

## Patentansprüche

1. Laryngoskop, mit einem Laryngoskopspatel (12), einem Handgriff (18) sowie mit einer Beleuchtungseinrichtung (20) zum Ausleuchten des Mund- bzw. Rachenraumes, wobei die Beleuchtungseinrichtung (20) zumindest ein erstes Licht abstrahlendes Element (22) aufweist, das am Laryngoskopspatel (12) angeordnet ist und im Betrieb einen Lichtkegel (28) mit einem Öffnungswinkel (30) abstrahlt, und wobei die Beleuchtungseinrichtung (20) zumindest ein zweites Licht abstrahlendes Element (24) aufweist, das ebenfalls am Laryngoskopspatel (12) angeordnet ist, **dadurch gekennzeichnet, dass** das zumindest eine zweite Licht abstrahlende Element (24) einen zweiten Lichtkegel (32) mit einem zweiten Öffnungswinkel (34) abstrahlt, der größer ist als der Öffnungswinkel (30) des Lichtkegels (28) des ersten Licht abstrahlenden Elements (22), und dass die Licht abstrahlenden Elemente (22, 24) so angeordnet sind, dass die Lichtkegel (28, 32) teilweise einander überlagert sind.

2. Laryngoskop nach Anspruch 1, **dadurch gekennzeichnet, dass** eine erste Hauptabstrahlrichtung (36) des ersten Licht abstrahlenden Elements (22) parallel zu einer zweiten Hauptabstrahlrichtung (38) des zweiten Licht abstrahlenden Elements (24) ist.

3. Laryngoskop nach Anspruch 1 oder 2, **dadurch gekennzeichnet, dass** der zweite Öffnungswinkel (34) um etwa das 1,5-fache bis 4- fache größer ist als der erste Öffnungswinkel (30).

4. Laryngoskop nach einem der Ansprüche 1 bis 3, **dadurch gekennzeichnet, dass** der erste Öffnungswinkel (30) im Bereich von etwa 10° bis etwa 30° und der zweite Öffnungswinkel (34) im Bereich von etwa 40° bis etwa 70° liegt.

5. Laryngoskop nach einem der Ansprüche 1 bis 4, **dadurch gekennzeichnet, dass** das erste Licht abstrahlende Element (22) und das zumindest eine zweite Licht abstrahlende Element (24) Lichtquellen sind.

6. Laryngoskop nach einem der Ansprüche 1 bis 5, **dadurch gekennzeichnet, dass** das erste Licht abstrahlende Element (22) und das zumindest eine zweite Licht abstrahlende Element (24) jeweils zumindest eine Leuchtdiode, insbesondere eine Weißlicht-Leuchtdiode aufweisen.

7. Laryngoskop nach Anspruch 6, **dadurch gekennzeichnet, dass** für die erste Leuchtdiode und die zumindest eine zweite Leuchtdiode eine gemeinsame Spannungsversorgung (50) vorgesehen ist.

8. Laryngoskop nach Anspruch 7, **dadurch gekennzeichnet, dass** die Spannungsversorgung (50) zumindest eine im Handgriff (18) angeordnete Batterie (51, 53) aufweist.

9. Laryngoskop nach Anspruch 7 oder 8, **dadurch gekennzeichnet, dass** für die zumindest eine Batterie (51, 53) eine Batteriespannung-Überwachungsschaltung (54) vorhanden ist.

10. Laryngoskop nach Anspruch 9, **dadurch gekennzeichnet, dass** die Batteriespannung-Überwachungsschaltung (54) ein Signal erzeugt, wenn die Batteriespannung einen vorgegebenen Mindestwert unterschreitet, wobei das Signal von zumindest einer der zwei Leuchtdioden erzeugt wird.

## Claims

1. A laryngoscope, comprising a laryngoscope spatula (12), a handle (18), and an illumination device (20) for illuminating the oral or pharyngeal cavity, the illumination device (20) having at least a first light-radiating element (22), which is arranged on the laryngoscope spatula (12) and, when operating, radiates a cone of light (28) with an angle of beam spread (30), and wherein the illumination device (20) has at least a second light-radiating element (24), which is likewise arranged on the laryngoscope spatula (12), **characterized in that** the at least one second light-emitting element (24) radiates a second cone of light (32) with a second angle of beam spread (34) which is greater than the angle of beam spread (30) of the cone of light (28) of the first light-radiating element (22), and that the light-radiating elements (22, 24) are arranged in such a way that the cones of light (28, 32) partially overlap each other.

2. The laryngoscope of Claim 1, **characterized in that** a first principal direction of radiation (36) of the first light-radiating element (22) is parallel to a second principal direction of radiation (38) of the second light-radiating element (24).

3. The laryngoscope of Claim 1 or 2, **characterized in that** the second angle of beam spread (34) is greater than the first angle of beam spread (30) by a factor of approximately 1.5 to 4.

4. The laryngoscope of any one of Claims 1 through 3, **characterized in that** the first angle of beam spread (30) ranges from roughly 10° to roughly 30° and the second angle of beam spread (34) ranges from roughly 40° to roughly 70°.

5. The laryngoscope of any one of Claims 1 through 4, **characterized in that** the first light-radiating element (22) and the at least one second light-radiating element (24) are light sources.

6. The laryngoscope of any one of Claims 1 through 5, **characterized in that** the first light-radiating element (22) and the at least one second light-radiating element (24) each have at least one light-emitting diode, in particular a white light-emitting diode.

7. The laryngoscope of Claim 6, **characterized in that** the first light-emitting diode and the at least one second light-emitting diode have a common power supply (50).

8. The laryngoscope of Claim 7, **characterized in that** the power supply (50) comprises at least one battery (51, 53) arranged in the handle (18).

9. The laryngoscope of Claim 7 or 8, **characterized in that** there is a battery-voltage monitoring circuit (54) for the at least one battery (51, 53).

10. The laryngoscope of Claim 9, **characterized in that** the battery-voltage monitoring circuit (54) generates a signal if the battery voltage drops below a predetermined minimum level, the signal being generated by at least one of the two light-emitting diodes.

## Revendications

1. Laryngoscope, comportant une lame (12) du laryngoscope, un manche (18), ainsi qu'un dispositif d'éclairage (20) pour éclairer la zone buccale et du pharynx, le dispositif d'éclairage (20) comportant au moins un élément (22), qui émet une première lumière et qui est disposé sur la lame (12) du laryngoscope et, en cours de fonctionnement, émet un cône de lumière (28) avec un angle d'ouverture (30), et le dispositif d'éclairage (20) comportant au moins un deuxième élément (24), qui émet une deuxième lumière et qui est disposé également sur la lame (12) du laryngoscope, **caractérisé en ce que** ledit au moins un deuxième élément (24), émettant une lumière, émet un deuxième cône de lumière (32) avec un deuxième angle d'ouverture (34), qui est plus grand que l'angle d'ouverture (30) du cône de lumière (28) du premier élément (22) émettant une lumière, et **en ce que** les éléments (22, 24) émettant de la lumière sont disposés de telle sorte que les cônes de lumière (28, 32) se chevauchent partiellement l'un l'autre.

2. Laryngoscope selon la revendication 1, **caractérisé en ce qu'**une première direction d'émission principale (36) du premier élément (22) émettant de la lumière est parallèle à une deuxième direction d'émission principale (38) du deuxième élément (24) émettant de la lumière.

3. Laryngoscope selon la revendication 1 ou 2, **caractérisé en ce que** le deuxième angle d'ouverture (34) est supérieur de 1,5 fois à 4 fois environ au premier angle d'ouverture (30).

4. Laryngoscope selon l'une quelconque des revendications 1 à 3, **caractérisé en ce que** le premier angle d'ouverture (30) se situe dans la plage de 10° environ à 30° environ, et le deuxième angle d'ouverture (34) se situe dans la plage de 40° environ à 70° environ.

5. Laryngoscope selon l'une quelconque des revendications 1 à 4, **caractérisé en ce que** le premier élément (22) émettant de la lumière et ledit au moins un deuxième élément (24) émettant de la lumière sont des sources lumineuses.

6. Laryngoscope selon l'une quelconque des revendications 1 à 5, **caractérisé en ce que** le premier élément (22) émettant de la lumière et ledit au moins un deuxième élément (24) émettant de la lumière comportent respectivement au moins une diode électroluminescente, en particulier une diode électroluminescente à lumière blanche.

7. Laryngoscope selon la revendication 6, **caractérisé en ce qu'**une alimentation de tension (50) commune est prévue pour la première diode électroluminescente et ladite au moins une deuxième diode électroluminescente.

8. Laryngoscope selon la revendication 7, **caractérisé en ce que** l'alimentation en tension (50) comporte au moins une pile (51, 53) disposée dans le manche (18).

9. Laryngoscope selon la revendication 7 ou 8, **caractérisé en ce qu'**un circuit de surveillance de la tension de la pile (54) est présent pour ladite au moins une pile (51, 53).

10. Laryngoscope selon la revendication 9, **caractérisé en ce que** le circuit de surveillance de la tension de la pile (54) délivre un signal lorsque la tension de la pile est inférieure à une valeur minimale prédéfinie, le signal étant généré par au moins l'une des deux diodes électroluminescentes.
